(19) Europäisches Patentamt
European Patent Office
Office européen des brevets

(11) Veröffentlichungsnummer: **0 392 464 B1**

(12) **EUROPÄISCHE PATENTSCHRIFT**

(45) Veröffentlichungstag der Patentschrift: **05.07.95**

(21) Anmeldenummer: **90106861.9**

(22) Anmeldetag: **10.04.90**

(51) Int. Cl.6: **C07C 69/54**, C07C 233/05, C07C 233/20, C07C 235/06, C07C 67/08, C07C 231/02, C08F 120/28, C08F 120/68, C08F 120/70, C08L 33/04, C08L 33/24

# (54) Amphiphile Monomere und Polymere und Film aus mindestens einer monomolekularen Schicht daraus.

(30) Priorität: **12.04.89 DE 3911929**

(43) Veröffentlichungstag der Anmeldung:
**17.10.90 Patentblatt 90/42**

(45) Bekanntmachung des Hinweises auf die Patenterteilung:
**05.07.95 Patentblatt 95/27**

(84) Benannte Vertragsstaaten:
**DE**

(56) Entgegenhaltungen:
**DE-A- 3 843 194**

**CHEMICAL ABSTRACTS, Band 86, No. 9, 28. Februar 1977, Columbus, Ohio, USA S.I.SADYKH-ZADE et al. "Synthesis of aliphatic mixed diethylene glycol esters of acrylic and methacrylic acids" Seite 396, abstract-Nr. 54 960g**

(73) Patentinhaber: **HOECHST AKTIENGESELLSCHAFT**

**D-65926 Frankfurt (DE)**

(72) Erfinder: **Lupo, Donald, Dr.**
**Am Holderbusch 28**
**D-6239 Eppstein / Taunus (DE)**
Erfinder: **Prass, Werner, Dr.**
**Bahnstrasse 19**
**D-6500 Mainz (DE)**
Erfinder: **Scheunemann, Udo, Dr.**
**Feldbergstrasse 12**
**D-6237 Liederbach (DE)**

EP 0 392 464 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

CHEMICAL ABSTRACTS, Band 103, Nr. 3, 22. Juli 1985, Columbus, Ohio, USA R.ELBERT et al. "Hydrophilic spacer groups in polymerizable lipids: formation of biomembrane models from bulk polymerized lipids" Seite 285, abstract-Nr. 19212n

CHEMICAL ABSTRACTS, Band 106, Nr. 8, 23. Februar 1987, Columbus, Ohio, USA A.LASCHEWSKY et al. "Self-organization of polymeric lipids with hydrophilic spacers in side groups and main chain: investigation in monolayers and multilayers" Seite 5, abstract-Nr. 50 714p

## Beschreibung

Die Erfindung bezieht sich auf spezielle amphiphile Monomere mit langen Alkylketten und deren Polymere, auf einen Film aus mindestens einer monomolekularen Schicht dieser Moleküle auf einem festen Schichtträger (= sogenannte Schichtelemente) und Verfahren zur Herstellung der Monomeren, der Polymeren und der Schichtelemente.

Zur Herstellung von geordneten Schichten organischer Polymerer mit langkettigen Seitengruppen wird überwiegend das Langmuir-Blodgett (LB)-Verfahren benutzt. Bei diesem Verfahren werden Moleküle auf einer Wasseroberfläche gespreitet und durch Verkleinerung der Fläche pro Molekül die langen Alkylseitengruppen parallel angeordnet. Bei konstantem Schub werden die Moleküle durch Ein- und Austauchen auf ein Substrat aufgezogen. Pro Tauchgang wird dabei eine monomolekulare Schicht unter Erhalt ihrer Ordnung übertragen.

Für den Aufbau von LB-Filmen verwendet man amphiphile Moleküle, d.h. Moleküle, die ein hydrophiles Ende (einen "Kopf") und eine hydrophobes Ende (einen "Schwanz") haben. Um eine höhere Stabilität der LB-Filme zu erreichen, wurden auch schon polymere LB-Filme hergestellt.

Hierzu wurden einmal ungesättigte Amphiphile nach der Herstellung des Films polymerisiert. Man hat aber auch schon unmittelbar organische Polymere mit langen Alkylseitenketten zur Schichtherstellung eingesetzt (Internationale Patentanmeldung WO83/03165 und R. Elbert, A. Laschewsky und H. Ringsdorf, J.Am.Chem.Soc. 107, 4134-4141 (1985)). Bei beiden Arten von polymeren Filmen treten jedoch mehr Fehlstellen als bei monomeren Filmen auf.

Bei der Polymerisation in der Schicht kommt es in nahezu allen Fällen zu einer Kontraktion in der Schicht, mit der Ausbildung von Fehlstellen. Bei Filmen aus Copolymeren, die ein hydrophobes Comonomer mit zwei langkettigen Alkylgruppen aufweisen, wie von A. Laschewsky, H. Ringsdorf, G. Schmidt und J. Schneider in J. Am.Chem.Soc. 109, 788-796 (1987) beschrieben, können durch das statistisch eingebaute hydrophile Comonomere unterschiedlich große hydrophile Schleifen entstehen, die zu Ungleichmäßigkeiten in der Schichtdicke führen.

Regelmäßige LB-Schichten aus Homopolymeren lassen sich im allgemeinen nur dann aufbauen, wenn die Alkylseitenkette und die Polymerhauptkette durch ein hydrophiles, flexibles Segment, den "hydrophilen Spacer", getrennt werden. Die von R. Elbert, A. Laschewsky und H. Ringsdorf in J.Am.Chem.Soc. 107, 4134-4141 (1985) beschriebenen Monomere sind jedoch als zweikettige Amphiphile nur mit relativ großem synthetischem Aufwand herstellbar. Bei Homopolymeren mit zwei langkettigen Alkylgruppen pro Monomer-Einheit kommt es, nach eigenen Untersuchungen, wegen des großen Raumbedarfs der beiden Alkylgruppen nicht zu einer vollständigen Entkopplung durch die "hydrophilen Spacer".

In DE-A 3843194 wurden bereits Filme aus Polymeren, die aus gemischtkettigen, amphiphilen, in mehreren Synthesestufen zugänglichen Monomeren herstellbar sind, vorgeschlagen.

Es bestand nun die Aufgabe, weitere, synthetisch einfacher zugängliche Monomere herzustellen, deren Homopolymeren eine gute Entkopplung der Ordnungstendenzen von Alkylseitenketten und Polymerhauptketten erlauben, sich besonders gut auf Schichtträger übertragen lassen und einen festeren Film bilden.

Die vorliegende Erfindung löst diese Aufgabe. Sie beruht auf der Synthese von einkettigen amphiphilen Monomeren, deren unpolarer Rest in einfacher Weise mit langkettigen Alkohlen bzw. Aminen eingebracht wird. Durch die einkettigen Amphiphilen ist es möglich, einfacher zu synthetisierende, relativ kurze hydrophile Spacer zu verwenden, um die Entkopplung der Ordnungstendenzen von Polymerhauptkette (Tendenz zum Polymerknäuel) und Alkylseitenkette (Tendenz zur Kristallisation) bereits in den Homopolymeren zu realisieren. Die Monomerbausteine haben folgende allgemeine Struktur (Formel I oder Formel II):

$$CH_2 = \overset{\overset{\displaystyle R^1}{|}}{C} - \overset{\overset{\displaystyle O}{\|}}{C} - O(-CH_2-CH_2-O)_n - \overset{\overset{\displaystyle O}{\|}}{C} - X - \overset{\overset{\displaystyle O}{\|}}{C} - R^2 \qquad (I)$$

$$CH_2 = \overset{\overset{\displaystyle R^1}{|}}{C} - \overset{\overset{\displaystyle O}{\|}}{C} - Y(-CH_2-CH_2-O)_1 - CH_2 - CH_2 - Y - \overset{\overset{\displaystyle O}{\|}}{C}(-CH_2)_m - CH_3, \qquad (II)$$

wobei
X = $(CH_2)_n$ oder $(CH_2 - O - CH_2)_n$,
Y = O oder NH,
1 eine ganze Zahl zwischen 0 und 10, m eine ganze Zahl zwischen 13 und 26, n eine ganze Zahl zwischen 1 und 20,
$R^1$ = H, $CH_3$, Cl, CN, F oder Br,
und $R^2$ ein O-Alkyl-Rest oder ein NH-Alkyl-Rest mit einer Alkylkette von mindestens 8 Kohlenstoffatomen, bevorzugt n-Alkyl mit 12-22 C-Atomen, ist.

Aus Ucenye Zap. Azerb. Un-t. Ser. Chim. Nauk, (1975), Heft 3-4, S. 77-80 (vgl. Chem. Abs. 86 (9) Abs. 54 960 g) ist die Synthese von Diethylenglycolmono(meth)acrylsäureester-monofettsäureester bekannt, wobei die Alkylgruppen der Fettsäuren 4 bis 11 C-Atome lang sind. Die Herstellung von Polymeren wird nicht beschrieben; es wird lediglich empfohlen, die Ester mit Hydrochinon zu versetzen, um eine Polymerisation zu verhindern.

Die Monomeren der allgemeinen Formel (I) lassen sich erstens durch Umsetzung einer Dicarbonsäure der allgemeinen Formel (III)

HOOC - X - COOH (III)

oder eines aktivierten Derivates dieser Dicarbonsäure, wie beispielsweise eines Säureanhydrides, Säureesters oder Säurehalogenides, mit einem ungesättigten Alkohol der allgemeinen Formel (IV)

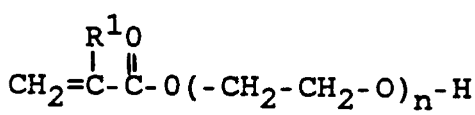
(IV)

zum Monoester und anschließender Kondensation mit einem langkettigen Alkohol bzw. primären Amin der allgemeinen Formel $R^2H$ herstellen.

Sie lassen sich aber auch durch die umgekehrte Reaktionsfolge darstellen, d.h. durch Umsetzung der Dicarbonsäure der Formel (III) oder eines ihrer reaktiven Derivate mit einem langkettigen Alkohol bzw. primären Amin $R^2H$ mit nachfolgender Veresterung des Zwischenproduktes mit dem ungesättigten Alkohol der Formel (IV).

Die Monomeren der allgemeinen Formel (II) werden durch Umsetzung einer Carbonsäure (V)

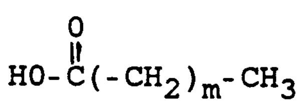
, (V)

oder eines reaktiven Carbonsäurederivates dieser Carbonsäure mit einem Alkohol bzw. primären Amin der allgemeinen Formel (VI),

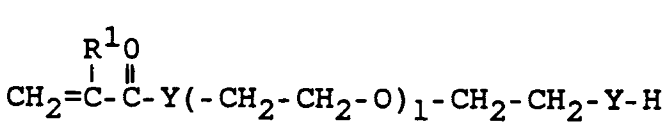
(VI)

vorzugsweise in Anwesenheit eines tertiären Amins synthetisiert. Als reaktive Carbonsäurederivate können beispielsweise Carbonsäurechlorid oder Carbonsäureanhydrid eingesetzt werden.

Zur Herstellung der erfindungsgemäßen Polymeren werden diese Monomeren homopolymerisiert oder auch mit anderen Monomeren copolymerisiert. Dabei können auch mehrere andere Monomere einpolymerisiert werden. Die Comonomeren können ebenfalls ungesättigte Monomere mit langen Alkylketten, die mindestens 8 C-Atome aufweisen, darstellen, beispielsweise Verbindungen der allgemeinen Formel VII

$$CH_2=\overset{R^1}{\overset{|}{C}}-\overset{O}{\overset{\|}{C}}-Y-(CH_2)_pCH_3 \qquad (VII)$$

wobei p eine ganze Zahl von 7 bis 21 bedeutet, oder andere Monomere nach Formel (I) oder (II) oder Monomeren wie sie in den obengenannten Publikationen und der DE-A 38 431 904 beschrieben sind. Auf diese Literaturstellen wird daher ausdrücklich Bezug genommen.

Als Comonomere können aber auch Vinylmonomere, die eine hydrophile Gruppe enthalten und vorzugsweise wasserlöslich sind, wie z.B. Itaconsäure, Fumarsäure, Maleinsäure, Acryl-, Cyanoacryl- und Methacrylsäure oder Derivate davon verwendet werden. Die erfindungsgemäßen Copolymere werden ausgehend von mindestens 10 Gew.-%, vorzugsweise mindestens 20 Gew.-%, eines Monomeren der allgemeinen Formel (I) oder (II) hergestellt. Die Polymerisation wird vorzugsweise radikalisch durchgeführt, insbesondere unter Zusatz eines Radikalbildners. Als Radikalbildner finden beispielsweise Azo-bis-isobutyronitril oder Peroxide Verwendung. Durch Zusatz von Reglern (z.B. Mercaptane wie Octadecanthiol) lassen sich Polymere mit kleiner Molmasse gewinnen. Die erfindungsgemäßen Polymere weisen schon ab einer Molmasse von ca. 5000 Dalton eine gute Eignung zur Herstellung eines Schichtelementes auf.

Zur Herstellung der erfindungsgemäßen Filme werden die organischen Polymeren oder Mischungen, die die erfindungsgemäßen polymeren, vorzugsweise zu 10 - 100 Gew-%, enthalten, in einem im wesentlichen flüchtigen, mit Wasser nicht mischbaren Lösungsmittel gelöst und auf die Oberfläche einer wäßrigen Lösung in einer Filmwaage gebracht (=gespreitet). Aus der Abmessung der Oberfläche, dem Spreitvolumen und der Konzentration der Lösung wird die mittlere Fläche pro Repetiereinheit berechnet. Phasenübergänge bei der Kompression der Moleküle lassen sich in der Schub-Flächen-Isotherme verfolgen.

Die Moleküle werden mit einer Barriere zusammengeschoben, wobei die Alkylketten bei wachsender Oberflächendichte im wesentlichen senkrecht zur Grenzschicht orientiert werden.

Während der Kompression entsteht durch Selbstorganisation der Moleküle an der Grenzschicht ein hochgeordneter monomolekularer Film, dessen konstante Schichtdicke im wesentlichen durch die Kettenlänge der Alkylseitenketten der Polymeren und deren Tiltwinkel (das ist der Winkel, um den die Molekülketten auf der Wasseroberfläche gegen die Normale gekippt sind) bestimmt wird. Die typische Dicke eines solchen Films liegt bei 2 - 3 nm.

Der Film wird bei konstantem Schub durch Eintauchen oder Austauchen eines geeigneten Trägers unter Erhalt der Ordnung von der Wasseroberfläche abgenommen.

Als Subphase für die Monofilmherstellung dienen meist Wasser oder wäßrige Lösungen. Es sind aber auch andere Flüssigkeiten mit hoher Oberflächenspannung, wie z. B. Glyzerin, Glykol, Dimethylsulfoxid, Dimethylformamid oder Acetonitril verwendbar.

Als Träger kommen beliebige feste, vorzugsweise dimensionsstabile Substrate aus unterschiedlichen Materialien in Betracht. Die als Schichtträger dienenden Substrate können beispielsweise transparent oder lichtdurchlässig, elektrisch leitend oder isolierend sein. Das Substrat kann hydrophob oder hydrophil sein. Die Oberfläche eines an sich hydrophilen Substrats, auf die die LB-Schicht aufgebracht wird, kann hydrophobiert sein. Die zu beschichtende Oberfläche des Substrats sollte möglichst rein sein, damit die Ausbildung einer dünnen, geordneten Schicht nicht gestört wird. Insbesondere die Anwesenheit von oberflächenaktiven Stoffen auf der zu beschichtenden Oberfläche der Substrate kann die Schichtherstellung beeinträchtigen. Es ist möglich, die als Schichtträger dienenden Substrate auf der zu beschichtenden Oberfläche vor dem Aufbringen der LB-Filme zunächst mit einer Zwischenschicht zu versehen, um z.B. die Haftung des Films auf dem Substrat zu verbessern.

Als Materialien für die Substrate können beispielsweise Metalle, wie etwa Gold, Platin, Nickel, Palladium, Aluminium, Chrom, Niob, Tantal, Titan, Stahl und dergleichen verwendet werden. Andere geeignete Materialien für die Substrate sind Kunststoffe, wie beispielsweise Polyester, etwa Polyethylenterephthalat oder Polybutylenterephthalat, Polyvinylchlorid, Polyvinylidenchlorid, Polytetrafluorethylen, Polystyrol, Polyethylen oder Polypropylen.

Gleichermaßen kommen auch Halbleiter, wie Silizium, Germanium oder Galliumarsenid oder auch Glas, Siliziumdioxid, keramische Werkstoffe oder Celluloseprodukte für die Substrate in Betracht. Die Oberfläche von Glas und anderen hydrophilen Substraten kann, sofern erforderlich, in an sich bekannter Weise durch Umsetzung mit Alkylsilanen oder Hexamethyldisilazan hydrophobiert werden. Die Auswahl der Substratmaterialien richtet sich in erster Linie nach dem Verwendungszweck der aus dem erfindungsgemäßen Film hergestellten Schichtelemente. Für optische Elemente werden in der Regel transparente, lichtdurchlässige

Substrate als Schichtträger eingesetzt. Werden die erfindungsgemäßen Schichtelemente beispielsweise in der Elektronik oder bei elektrochemischen Prozessen verwendet, dienen als Substrate insbesondere elektrisch leitfähige Materialien, wie Metalle oder Indium-Zinnoxid oder metallische Oberflächenschichten, beispielsweise auf Kunststoffolien oder Glas.

Die als Träger für die erfindungsgemäßen Filme dienenden Substrate können, je nach Verwendungszweck, beliebige Formen aufweisen. Beispielsweise können sie film-, folien-, platten-, bandförmig oder auch zylinderförmig sein oder unter anderen beliebigen Formen ausgewählt werden. Im allgemeinen wird es sich bei den Schichtträgern um flache, ebene Substrate handeln, wie z.B. Filme, Folien, Platten, Bänder und dergleichen. Die zu beschichtende Oberfläche der Substrate ist vorzugsweise glatt, wie es für die Herstellung von LB-Filmen üblich ist. Bei flachen, ebenen Substraten können die erfindungsgemäßen Filme auf eine oder beide Oberflächen des Substrats aufgebracht werden.

Die erfindungsgemäßen Polymeren zeichnen sich durch eine gute Herstellbarkeit einer Multischichtstruktur mit wenigen Defektstellen aus, wobei die Schichtstruktur eine gute Temperaturstabiltät aufweist.

Solche Filme auf Substraten eignen sich beispielsweise für optische Wellenleitersysteme oder zur Herstellung von Filtern für optische Zwecke. Aufgrund der geringen kritischen Oberflächenspannung sind die Filme auch zur Verbesserung der Reibungseigen schaften von Materialien, zur Herstellung von Schutzschichten sowie für weitere einschlägige Anwendungen geeignet.

Die Erfindung wird durch die nachfolgenden Beispiele näher erläutert.

**Beispiel 1: Synthese von Monomer 4**

$$CH_3-(CH_2)_{15}-NH-\overset{\overset{O}{\|}}{C}-CH_2-CH_2-\overset{\overset{O}{\|}}{C}-O-CH_2-CH_2-O-\overset{\overset{O}{\|}}{C}-\overset{\overset{CH_3}{|}}{C}=CH_2$$

6,0 g (60 mmol) Bernsteinsäureanhydrid und 4,7 g (60 mmol) Pyridin werden in 200 ml trockenem Toluol gelöst und bei 25 °C unter Rühren und Feuchtigkeitsausschluß eine Lösung von 6,5 g (50 mmol) Methacrylsäure-2-hydroxyethylester und 5 mg 2,6-Di-tert.-butyl-p-kresolin 50 ml trockenem Toluol innerhalb von 5 Minuten zudosiert. Nach beendeter Zugabe wird die Reaktionsmischung erhitzt und 16 Stunden lang bei 70-80 °C gerührt. Nach dem Abkühlen wird das Lösungsmittel im Vakuum entfernt und die erhaltene Flüssigkeit im Ölpumpenvakuum getrocknet.

Man erhält 9,26 g (80.5 %) einer wasserklaren Flüssigkeit.

$^1$H-NMR (100 MHz, $CDCl_3$): $\delta$ = 1,9 (t, 3H; $-CH_3$), 2,65 (s, 4H; $-CO-CH_2-CH_2-CO-$), 4,35 (s, 4H; $-O-CH_2-CH_2-O-$), 5,6, 6,1 (AB, 2H; $=CH_2$)

Zu einer Lösung von 9,66 g Hexadecylamin und 12,0 g (55 mmol) des oben hergestellten Bernsteinsäureesters in 250 ml trockenem Methylenchlorid wird bei 0 °C unter Rühren und Feuchtigkeitsausschluß eine Lösung von 10,32 g (50 mmol) Dicyclohexylcarbodiimid und 150 mg frisch umkristallisiertes N,N-Dimethylaminopyridin in 100 ml trockenem Methylenchlorid innerhalb von 20 Minuten zugesetzt. Man rührt 6 Stunden bei 0 °C, filtriert den ausgefallenen Niederschlag ab und entfernt das Lösungsmittel im Vakuum. Das Rohprodukt wird säulenchromatographisch gereinigt (Kieselgel Si60, Eluent: Hexan/Ethylacetat 1:1).

Man erhält 13,8 g (30,4 mmol, 76 %) eines weißen Pulvers, das bei 48 °C schmilzt.

$^1$H-NMR (100 MHz, $CDCl_3$): $\delta$ = 0,9 (t, 3H; $-CH_3$ Alkylkette), 1,1-1,5 (m, 28H, $-CH_2-$ Alkylkette), 1,9 (t, 3H; $-CH_3$ Methacrylsäure), 2,3-2,8 (m, 4H; $-CO-CH_2-CH_2-CO-$), 3,1-3,3 (m, 2H; $-CH_2-N$) 4,35 (s, 4H; $-O-CH_2-CH_2-O-$), 5,6, 6,1 (AB, 2H; $=CH_2$)

**Beispiel 2: Synthese von Monomer 7**

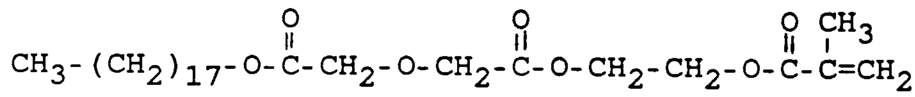

13,0 g (0,1 mol) Methacrylsäure-2-hydroxyethylester, 13,4 g Diglykoldisäure und 10 mg 2,6-Di-tert.-butyl-p-kresol, gelöst in 250 ml trockenem Tetrahydrofuran werden bei 0 °C unter Feuchtigkeitsausschluß vorgelegt und unter Rühren innerhalb von 10 Minuten eine Lösung von 20,6 g (0,1 mol) Dicyclohexylcarbodiimid und 250 mg frisch umkristallisiertes N,N-Dimethylaminopyridin in 50 ml trockenem THF zudosiert. Man rührt bei 0 °C noch zwei Stunden und läßt dann unter Rühren auf Raumtemperatur erwärmen. Am nächsten Morgen wird der entstandene Niederschlag abfiltriert, das Lösungsmittel im Vakuum entfernt. Das so erhaltene Rohprodukt wird säulenchromatographisch gereinigt (Kieselgel Si60, Eluent Chloroform/Methanol/Eisessig 100:15:1). Man erhält 18 g (73 mmol, 73 %) Reinprodukt.
$^1$H-NMR (100 MHz, $CDCl_3$): $\delta$ = 1,9 (m, 3H; $-CH_3$), 4,25 (s, 4H; $-CO-CH_2-O-CH_2-CO-$), 4,3-4,5 (m, 4H; $-O-CH_2-CH_2-O-$), 5,6, 6,1 (AB, 2H; $=CH_2$)

Zu einer Lösung von 13,5 g (50 mmol) Octadecanol und 13,5 g (50 mmol) des oben hergestellten Diglykolsäuremonoesters in 250 ml trockenem Methylenchlorid wird bei 0 °C unter Rühren und Feuchtigkeitsausschluß eine Lösung von 10,3 g (50 mmol) Dicyclohexylcarbodiimid und 150 mg frisch umkristallisiertes N,N-Dimethylaminopyridin in 100 ml trockenem Methylenchlorid innerhalb von 20 Minuten zugesetzt. Man rührt zwei Stunden bei 0 °C, filtriert den ausgefallenen Niederschlag ab wäscht die organische Phase mit Wasser, trocknet mit Natriumsulfat und entfernt das Lösungsmittel im Vakuum. Durch Umkristallisation aus Hexan wird nicht umgesetztes Octadecanol zurückgewonnen. Das so vorgereinigte Produkt wird säulenchromatographisch gereinigt (Kieselgel Si60, Eluent: Hexan/Ethylacetat 8:1). Man erhält in der zweiten Fraktion 4,5 g (9,0 mmol, 10 %) einer weißen wachsartigen Masse, die zwischen 36,5 und 37.5 °C schmilzt.
$^1$H-NMR (100 MHz, $CDCl_3$): $\delta$ = 0,9 (t, 3H; $-CH_3$ Alkylkette), 1,1-1,8 (m, 32H, $-CH_2$-Alkylkette), 1,9 (m, 3H; $-CH_3$ Methacrylsäure), 4,0-4,5 (m, 10H; $-CO-CH_2-O-CH_2-CO-$, $-CH_2-O-CO-$, $-O-CH_2-CH_2-O-$), 5,6, 6,1 (AB, 2H; $=CH_2$)

**Beispiel 3: Synthese von Monomer 3**

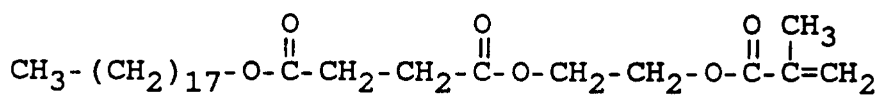

13,3 g (50 mmol) Octadecanol und 6,1 g (60 mmol) Triethylamin werden in 100 ml trockenem Toluol gelöst und bei 20 °C unter Rühren und Feuchtigkeitsausschluß eine Lösung von 9,0 g (90 mmol) Bernsteinsäureanhydrid in 200 ml trockenem Toluol innerhalb von 15 Minuten zudosiert. Nach beendeter Zugabe wird die Reaktionsmischung erhitzt und 40 Stunden unter Rückfluß gekocht. Nach dem Abkühlen wird die Reaktionslösung mit 300 ml 1 M HCl ausgeschüttelt, die organische Phase mit $MgSO_4$ getrocknet und das Lösungsmittel im Vakuum entfernt. Zur Reinigung des Rohprodukts wird anschließend je 1 Mal aus Hexan und Methanol umkristallisiert. Man erhält 10,70 g (85 %) eines weißen Pulvers, das zwischen 83 und 85 °C schmilzt.
$^1$H-NMR (100 MHz, $CDCl_3$): $\delta$=0,9 (t, 3H; $-CH_3$), 1,0-1,8 (m, 32H; $-CH_2$-Alkylkette), 2,65 (m, 4H; $-CO-CH_2-CH_2-CO-$), 4,05 (t, 2H; $-CH_2-O-$)

Zu einer Lösung von 5,49 g (42,4 mmol) 2-Hydroxyethylmethacrylat, 50 mg 2,6-Di-t-butyl-p-kresol und 10,0 g (27 mmol) des oben hergestellten Bernsteinsäureesters in 300 ml trockenem Methylenchlorid wird bei 0 °C unter Rühren und Feuchtigkeitsausschluß eine Lösung von 6,1 g (29,5 mmol) Dicyclohexylcarbodiimid und 130 mg frisch umkristallisiertes N,N-Dimethylaminopyridin in 100 ml trockenem Methylenchlorid innerhalb von 20 Minuten zugesetzt. Man rührt 1 Stunde bei 0 °C und läßt dann auf Raumtemperatur erwärmen. Am nächsten Morgen filtriert man den ausgefallenen Niederschlag ab, wäscht die Lösung mit

Wasser, trocknet mit $Na_2SO_4$ und entfernt das Lösungsmittel im Vakuum. Das Rohprodukt wird durch Umkristallisieren aus Ethanol gereinigt. Man erhält 8,59 g (17,8 mmol, 66 %) eines weißen Pulvers, das bei 36-37 °C schmilzt.
$^1$H-NMR (100 MHz, $CDCl_3$): $\delta$ = 0,9 (t, 3H; $-CH_3$ Alkylkette), 1,1-1,8 (m, 32H, $-CH_2$-Alkylkette), 1,9 (t, 3H; $-CH_3$ Methacrylsäure), 2,5-2,8 (m, 4H; $-CO-CH_2-CH_2-CO-$), 4,05 (t, 2H; $-CH_2-O-CO-$) 4,35 (s, 4H; $-O-CH_2-CH_2-O-$), 5,6, 6,1 (AB, 2H; $=CH_2$ Methacrylsäure)

**Beispiel 4: Synthese von Monomer 11**

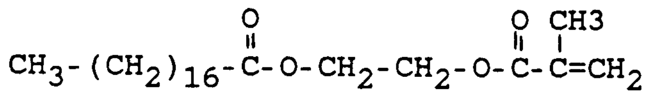

Zu einer Lösung von 10 g (33 mmol) Stearinsäurechlorid in 100 ml trockenem Methylenchlorid wird bei 5 °C unter Feuchtigkeitsausschluß innerhalb von 20 Minuten eine Lösung von 4,01 ml (4,3 g, 33 mmol) 2-Hydroxyethylmethacrylat, 5,55 ml (4,05 g, 40 mmol) Triethylamin und 20 mg 2,6-Di-tert.-butyl-p-kresol in 50 ml trockenem Methylenchlorid zudosiert. Nach beendeter Zugabe wird noch 30 Minuten bei 5 °C gerührt; anschließend läßt man auf 20 °C erwärmen und rührt noch zwei Stunden nach. Am nächsten Morgen wird das Reaktionsgemisch zwei Mal mit 1 M HCl ausgeschüttelt, die organische Phase mit Natriumsulfat getrocknet und das Lösungsmittel im Vakuum entfernt. Zur Reinigung wird noch je zwei Mal aus je 50 ml Methanol und Hexan umkristallisiert.
Man erhält 8,6 g (22 mmol, 66 %) einer wachsartigen weißen Masse.
$^1$H-NMR (100 MHz, $CDCl_3$): $\delta$ = 0,9 (t, 3H; $-CH_3$ Alkylkette), 1,5-1,8 (m, 30H; $-CH_2$-Alkylkette), 1,9-2,0 (m, 3H; $-CH_3$ Methacrylsäure), 2,2-2,5 (m, 2H; $-CH_2-CO-$), 4,3 (s, 4H; $-O-CH_2-CH_2-O-$), 5,6, 6,1 (AB, 2H; $=CH_2$)

**Beispiel 5: Radikalische Homopolymerisation von Monomer 4**

1 g des in Beispiel 1 hergestellten Monomeren werden in 20 ml Tetrahydrofuran gelöst und mit 7,0 mg Azo-bis-isobutyronitril versetzt. Die Lösung wird in einen Dreihalskolben mit Rückflußkühler (mit Gasableitungsrohr und Blasenzähler), Thermometer und Gaseinleitungsrohr gegeben und bei Raumtemperatur eine Stunde lang mit Stickstoff durchspült. Dann wird bis zum Rückfluß erhitzt (Innentemperatur: 65 °C) und 7 Stunden unter Rückfluß gekocht. Dabei wird die Reaktionsmischung ständig mittels Magnetrührer gerührt und mit Stickstoff gespült. Das Polymere wird durch Eingießen der Reaktionslösung in Methanol ausgefällt und abgesaugt. Man erhält 710 mg einer weißen feinkörnigen Masse, die in Hexan und Methanol unlöslich und in Tetrahydrofuran löslich ist. Eine Molmassenbestimmung mittels Gelpermeationschromatographie ergab ein Mw von 26 000 und ein Mn von 17 000 Dalton (Polystyrol-Eichung).

**Beispiel 6: Radikalische Homopolymerisation von Monomer 7**

1 g des in Beispiel 2 hergestellten Monomeren werden in 10 ml Tetrahydrofuran gelöst und mit 6,8 mg Azo-bis-isobutyronitril versetzt. Die Lösung wird in einen Dreihalskolben mit Rückflußkühler (mit Gasableitungsrohr und Blasenzähler), Thermometer und Gaseinleitungsrohr gegeben und bei Raumtemperatur eine Stunde lang mit Stickstoff durchspült. Dann wird bis zum Rückfluß erhitzt (Innentemperatur: 65 °C) und 6 Stunden unter Rückfluß gekocht. Dabei wird die Reaktionsmischung ständig mittels Magnetrührer gerührt und mit Stickstoff gespült. Das Polymere wird durch Eingießen der Reaktionslösung in Methanol ausgefällt und abgesaugt. Man erhält 590 mg einer weißen feinkörnigen Masse, die in Hexan und Methanol unlöslich und in Tetrahydrofuran löslich ist. Eine Molmassenbestimmung mittels Gelpermeationschromatographie ergab ein Mw von 26 000 und ein Mn von 18 000 Dalton (Polystyrol-Eichung).

**Beispiel 7: Radikalische Copolymerisation von Monomer 11 mit 2-Hydroxyethylmethacrylat**

2 g (5,04 mmol) des in Beispiel 4 hergestellten Monomeren und 0,66 g (5,07 mmol) 2-Hydroxyethylmethacrylat werden in 20 ml Tetrahydrofuran gelöst und mit 8,3 mg Azo-bis-isobutyronitril versetzt. Die Lösung wird in einen Dreihalskolben mit Rückflußkühler (mit Gasableitungsrohr und Blasenzähler), Thermometer und Gaseinleitungsrohr gegeben und bei Raumtemperatur eine Stunde lang mit Stickstoff durchspült.

Dann wird bis zum Rückfluß erhitzt (Innentemperatur: 65 °C) und 6 Stunden unter Rückfluß gekocht. Dabei wird die Reaktionsmischung ständig mittels Magnetrührer gerührt und mit Stickstoff gespült. Das Polymere wird durch Eingießen der Reaktionslösung in Methanol ausgefällt und abgesaugt. Um das Produkt von Restmonomer zu befreien wird es noch zwei Mal in Tetrahydrofuran gelöst und durch Eingießen in Methanol ausgefällt. Man erhält 1,4 g einer weißen feinkörnigen Masse, die in Hexan und Methanol unlöslich und in Tetrahydrofuran löslich ist. Eine Molmassenbestimmung mittels Gelpermeationschromatographie ergibt ein Mw von 51 000 und ein Mn von 27 000 Dalton (Polystyrol-Eichung). Aus der Elementaranalyse (66,9 % C, 10,2 % H) ergibt sich eine Zusammensetzung des Copolymeren von 1 Teil langkettig substituiertem Monomeren und 1,4 Teilen 2-Hydroxyethylmethacrylat.

**Beispiel 8: Schichtherstellung nach der Langmuir-Blodgett Methode**

Ein Glas-Objektträger (76 mm x 26 mm) wird nach folgendem Verfahren gereinigt:
Das Glas wird eine Stunde lang in eine 60 °C warme, frisch angesetzte Mischung aus vier Teilen konz. $H_2SO_4$ und einem Teil 30 %iger $H_2O_2$ gelegt, mit sauberem Wasser abgespült und 15 Minuten lang in einer Reinigungslösung (Extran/ AP 11, Konz. 2-4 g/l) bei 50 °C ultrabeschallt. Danach wird wieder gründlich mit sauberem Wasser abgespült und in einem warmen Luftstrom getrocknet. Anschließend erfolgt zur Hydrophobisierung eine Behandlung mit Hexamethyldisilazan-Dampf (10 Minuten bei 70 °C). Multischichten aus dem in Beispiel 5 hergestellten Polymeren werden nach dem Verfahren von Langmuir und Blodgett auf den Glasträger übertragen, indem in einer Langmuir-Filmwaage 250 cm3 einer Lösung von 5,1 mg des Polymeren in 10 $cm^3$ einer Mischung aus Methylenchlorid und Tetrahydrofuran 9:1 (v/v) auf einer wäßrigen Subphase bei einer Subphasentemperatur von 20 °C gespreitet werden. Durch Verkleinerung der monofilmbedeckten Wasseroberfläche wird der Schub auf 15 mN/m eingeregelt und bei diesem Wert konstant gehalten. Der Träger wird nun senkrecht von oben durch die Wasseroberfläche in die Filmwaage eingetaucht (Eintauchgeschwindigkeit: 20 mm/min) und nach einer kurzen Pause (10 sec.) am unteren Umkehrpunkt wieder herausgenommen (Austauchgeschwindigkeit: 10 mm/min) Sowohl beim Eintauch- als auch bei Austauchvorgang wird dabei eine Monolage auf den Träger übertragen. Durch mehrfache Wiederholung des Tauchvorgangs nach jeweils einer Minute Wartezeit am oberen Umkehrpunkt werden insgesamt 10 Doppelschichten übertragen. Die Übertragungsverhältnisse liegen bei 95 %. Es werden, auch bei Übertragung von 50 und mehr Monolagen optisch klare, transparente Schichten erhalten.

Nach der gleichen Verfahrensweise werden auch Schichten aus den in den Beispielen 6 und 7 hergestellten Polymeren erhalten. Die Übertragungsbedingungen hierbei sind:

| **Polymer aus Beispiel:** | **6** | **7** |
|---|---|---|
| Subphasentemperatur: | 30 °C | 30 °C |
| Schub: | 30 mN/m | 25 mN/m |
| Übertragungsverhältnis: | 80 % | 90 % |

**Beispiel 9: Ellipsometrische Schichtdicken- und Brechungsindexmessungen**

Ein Siliziumplättchen (40 mm x 10 mm) wird aus einem Siliziumwafer heraußgeschnitten und wie folgt gereinigt:

1. 1 Stunde Behandlung in einer heißen (60 °C), frisch angesetzten Mischung aus einem Teil 30 %iger $H_20_2$ und vier Teilen konz. $H_2S0_4$. Anschließend wird mit saüberem Wasser abgespült.
2. 30 Sekunden Eintauchen in $NH_4F$ gepufferte HF-Lösung und anschließend wieder mit saüberem Wasser abspülen. Nach dieser Behandlung sind die Siliziumplättchen hydrophob (Kontaktwinkel zu Wasser: 75 °).

Schichten der in den Beispielen 5, 6 und 7 hergestellten Polymeren werden nach dem Verfahren von Langmuir und Blodgett unter den gleichen Bedingungen wie in Beispiel 8 auf das Siliziumplättchen übertragen. Es werden jeweils Proben mit 10, 30, 50 und 70 Monolagen der einzelnen Polymeren hergestellt und ellipsometrisch die Schichtdicken und der Brechungsindex der LB-Filme gemessen.

| **Ergebnisse der Messungen:** | | | |
|---|---|---|---|
| Polymere aus Beispiel: | 5 | 6 | 7 |
| Brechungsindex bei 633 nm:<br>Schichtdicke in nm/Monolage: | 1,51<br>1,42 | 1,488<br>1,92 | 1,499<br>2,2 |

**Beispiel 10: Messungen der thermischen Stabilität**

Siliziumplättchen (40 mm x 10 mm) werden aus einem thermisch oxidierten Siliziumwafer (Dicke der Oxidschicht: 160 nm) herausgeschnitten und eine Stunde lang bei 60 °C in eine frisch zübereitete Mischung aus einem Teil 30 %iger $H_2O_2$ und vier Teilen konz. $H_2SO_4$ gelegt. Nach gründlichem Abspülen mit saüberem Wasser wird das Plättchen 15 Minuten lang bei 50 °C in einem Ultraschallbad mit alkalischem Reinigungsbad (Extran/ AP 11, Konz. 2-4 g/l) behandelt, mit sauberem Wasser gründlich abgespült und in einem warmen Luftstrom getrocknet. Danach erfolgt zur Hydrophobisierung eine Behandlung mit Hexamethyldisilazan-Dampf (10 Minuten bei 70 °C).

Die Beschichtung nach der LB-Methode mit jeweils 8 Monolagen erfolgt mit den in den Beispielen 5, 6 und 7 hergestellten Polymeren wie in Beispiel 8 beschrieben.

Der beschichtete Träger wird in einer speziellen Apparatur mit linearem Temperaturgradienten (0.5 °C/sec) aufgeheizt. Während des Aufheizvorgangs wird die Dicke der LB-Schicht anhand der Intensität eines von der Probe reflektierten, senkrecht polarisierten Laserstrahls (633 nm) gemessen. Die Temperatur, bei der die erste Änderung der Schichtdicke erfolgt, beträgt bei Schichten aus dem in Beispiel 5 hergestellten Polymeren 195 °C, bei Schichten aus dem in Beispiel 6 hergestellten Polymeren 85 °C und bei Schichten aus dem in Beispiel 7 hergestellten Polymeren 195 °C. (Zum Vergleich: Bei LB-Schichten aus 22-Tricosensäure beträgt diese Temperatur 70 °C).

**Beispiel 11: Messungen der kritischen Oberflächenspannung**

Siliziumplättchen (40 mm x 10 mm) werden wie in Beispiel 9 gereinigt und wie in Beispiel 8 jeweils mit acht Monolagen der in den Beispielen 5, 6 und 7 hergestellten Polymeren beschichtet.

Auf die Oberfläche der übertragenen Schichten werden Flüssigkeitstropfen einer Reihe von n-Alkanen ($C_9H_{20}$ -$C_{16}H_{34}$) gebracht und die Kontaktwinkel der Flüssigkeitstropfen mit der Oberfläche gemessen. Aus diesen Kontaktwinkeln wird nach dem Verfahren von Zisman die kritische Oberflächenspannung bestimmt. Es ergeben sich folgende Werte:

| Polymeres aus Beispiel | krit. Oberflächenspannung in mN/m |
|---|---|
| 5<br>6<br>7 | 25,1<br>22,8<br>25,2 |
| (Zum Vergleich: Bei einer Polyethylen- Oberfläche ergibt sich bei dieser Messung ein Wert von 31 mN/m). | |

**Beispiel 12:**

Die in der nachfolgenden Tabelle zusätzlich aufgeführten Monomere wurden nach Verfahren hergestellt, die analog zu den Synthesen der Beispiele 1 bis 4 sind.

**Tabelle der synthetisierten Monomeren:**

| Verb. Nr. | Strukturformel |
|---|---|
| 1 | 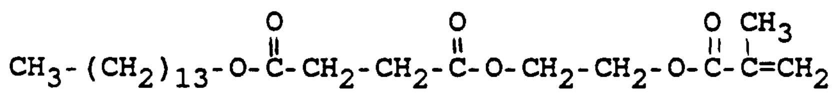 |
| 2 | 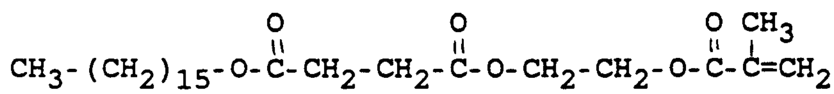 |
| 3 | 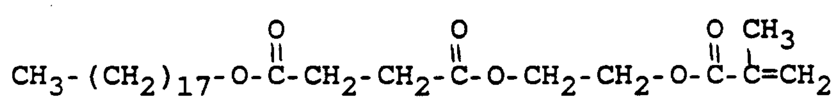 |
| 4 | 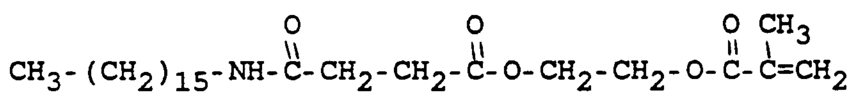 |
| 5 | 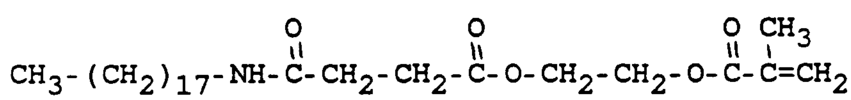 |
| 6 | 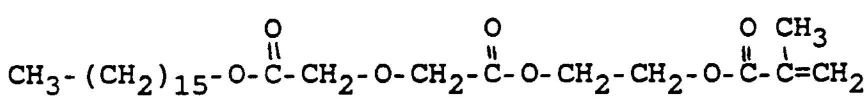 |
| 7 | 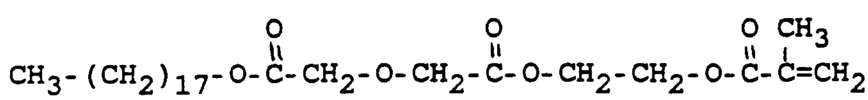 |
| 8 | 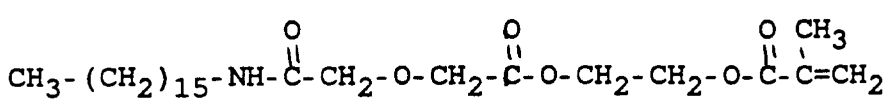 |

**Tabelle der synthetisierten Monomeren: (Fortsetzung)**

| Verb. Nr. | Strukturformel |
|---|---|
| 9 | 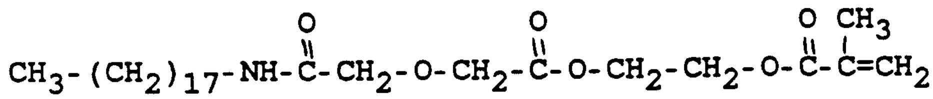 |
| 10 | 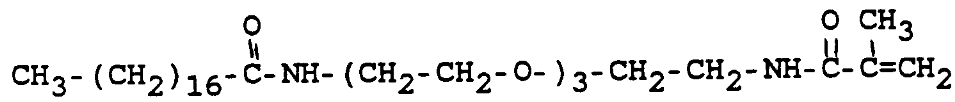 |
| 11 | 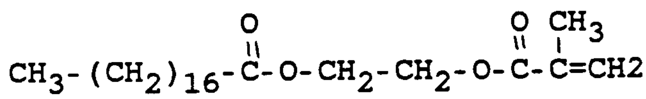 |

## Patentansprüche

1. Amphiphiles Monomer der allgemeinen Formeln (I) oder (II)

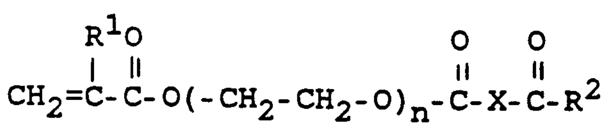 (I)

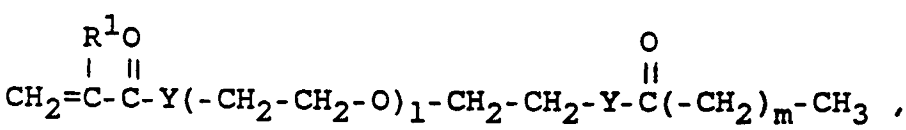 , (II)

wobei
$X = (CH_2)_n$ oder $(CH_2\text{-}O\text{-}CH_2)_n$ ,
Y = O oder NH ,
l eine ganze Zahl zwischen 0 und 10, m eine ganze Zahl zwischen 13 und 26, bevorzugt zwischen 13 und 21, n eine ganze Zahl zwischen 1 und 10,
$R^1$ = H, $CH_3$, Cl, CN, F oder Br,
und $R^2$ ein O-Alkyl-Rest oder ein NH-Alkyl-Rest mit einer Alkylkette von mindestens 8 Kohlenstoffatomen, bevorzugt n-Alkyl mit 12 - 22 C-Atomen, ist.

**2.** Verfahren zur Herstellung eines Monomeren der allgemeinen Formel (I)

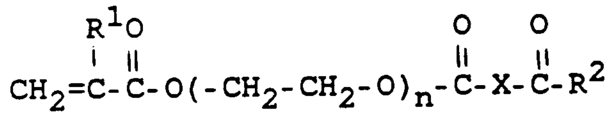

(I)

(wobei X, n, $R^1$ und $R^2$ die in Anspruch 1 angegebene Bedeutung haben)
dadurch gekennzeichnet, daß man eine Dicarbonsäure der allgemeinen Formel (III)

HOOC-X-COOH (III)

oder ein aktiviertes Derivat dieser Dicarbonsäure mit einem ungesättigten Alkohol der allgemeinen Formel (IV) zur Reaktion bringt

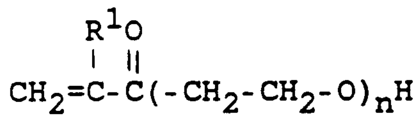

(IV)

und den dabei entstandenen Monoester mit einem langkettigen Alkohol $R^2H$ bzw. primären Amin $R^2H$ weiter umsetzt.

**3.** Verfahren zur Herstellung eines Monomeren der allgemeinen Formel (I), dadurch gekennzeichnet, daß man zunächst eine Dicarbonsäure der allgemeinen Formel (III)

HOOC-X-COOH (III)

oder ein aktiviertes Derivat dieser Dicarbonsäure mit einem langkettigen Alkohol $R^2H$ bzw. primären Amin $R^2H$ zur Reaktion bringt und das dabei entstandene Produkt mit einem ungesättigten Alkohol der allgemeinen Formel (IV)

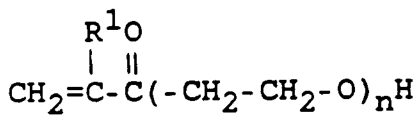

(IV)

weiter umsetzt,
wobei X, $R^1$ und n die in Anspruch 1 genannte Bedeutung haben.

**4.** Verfahren zur Herstellung eines Monomeren der allgemeinen Formel (II),

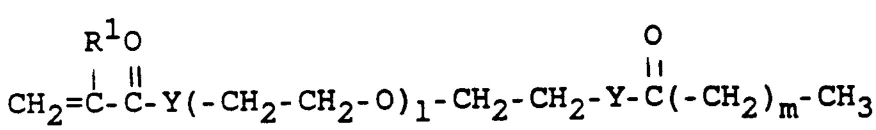

, (II)

(wobei l, m, $R^1$ und Y die in Anspruch 1 angegebene Beudeutung haben)
dadurch gekennzeichnet, daß man eine Carbonsäure der allgemeinen Formel (V) oder ein reaktives Carbonsäurederivat dieser Carbonsäure

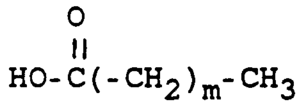

(V)

mit einem Alkohol bzw. Amin der allgemeinen Formel (VI),

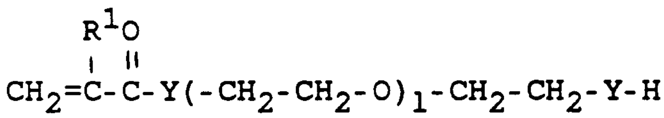

(VI)

vorzugsweise in Anwesenheit eines tertiären Amins umsetzt,

5. Homopolymer erhalten durch Polymerisation eines Monomeren nach Anspruch 1.

6. Copolymer erhalten durch Polymerisation eines oder mehrerer Monomere der Formeln (I) und/oder (II) nach Anspruch 1 sowie gegebenenfalls weiterer hydrophiler Co-monomere.

7. Verfahren zur Herstellung von Polymeren gemäß Ansprüchen 5 oder 6 durch Polymerisation von Monomeren, dadurch gekennzeichnet, daß man die Polymerisation unter Zusatz eines Radikalbildners vornimmt.

8. Schichtelement aus einem auf einem festen Träger aufgebrachten Film, bestehend aus mindestens einer monomolekularen Schicht einer amphiphilen Verbindung, dadurch gekennzeichnet, daß die Schicht ein Polymeres nach Anspruch 5 und/oder 6 enthält.

9. Verfahren zur Herstellung eines Schichtelementes gemäß Anspruch 8, dadurch gekennzeichnet, daß man mindestens ein Polymeres gemäß Ansprüchen 5 und/oder 6 in einem flüchtigen organischen, mit Wasser nicht mischbaren Lösungsmittel löst, die Lösung an der Grenzfläche Wasser/Luft spreitet, die entstehende Schicht nach Verdunsten des Lösungsmittels komprimiert und nach der Langmuir-Blodgett-Technik auf einen festen Träger überträgt.

10. Verwendung eines Films aus einem Polymeren nach einem der Ansprüche 5 und/oder 6 zur Vergütung von Oberflächen und zur Verminderung der Reibung zwischen Oberflächen.

11. Verwendung des Schichtelementes nach Anspruch 8 für optische Zwecke.

12. Verwendung eines Films aus einem Polymeren nach einem der Anspruche 5 oder 6 zur elektrischen Isolation.

**Claims**

1. An amphiphilic monomer of the general formulae (I) or (II)

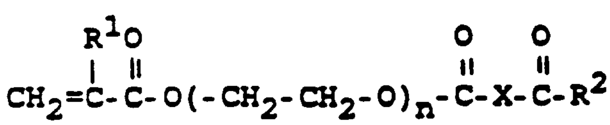

(I)

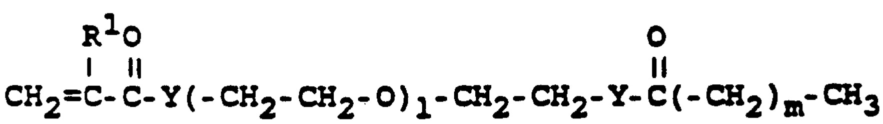

(II)

where
X is $(CH_2)_n$ or $(CH_2\text{-}O\text{-}CH_2)_n$,
Y is O or NH,
l is an integer from 0 to 10, m is an integer from 13 to 26, preferably from 13 to 21, n is an integer from 1 to 10,
$R^1$ is H, $CH_3$, Cl, CN, F or Br,
and $R^2$ is an O-alkyl radical or an NH-alkyl radical having an alkyl chain of at least 8 carbon atoms, preferably n-alkyl of 12-22 carbon atoms.

**2.** A process for preparing a monomer of the general formula (I)

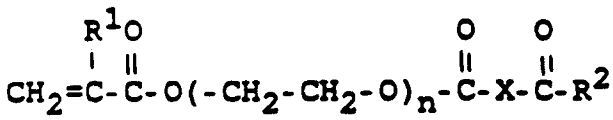 (I)

(where X, n, $R^1$ and $R^2$ are each as defined in claim 1), which comprises reacting a dicarboxylic acid of the general formula (III)

HOOC-X-COOH (III)

or an activated derivative thereof with an unsaturated alcohol of the general formula (IV)

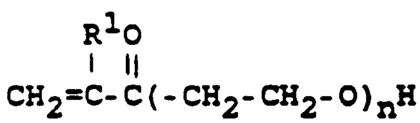 (IV)

and further reacting the resulting monoester with a long-chain alcohol $R^2H$ or primary amine $R^2H$.

**3.** A process for preparing a monomer of the general formula (I), which comprises first reacting a dicarboxylic acid of the general formula (III)

HOOC-X-COOH (III)

or an activated derivative thereof with a long-chain alcohol $R^2H$ or primary amine $R^2H$ and further reacting the resulting product with an unsaturated alcohol of the general formula (IV)

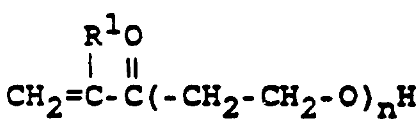 (IV)

where X, $R^1$ and n are each as defined in claim 1.

**4.** A process for preparing a monomer of the general formula (II)

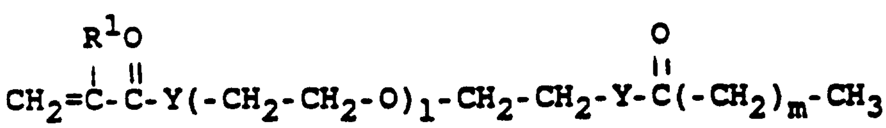 (II)

(where l, m, $R^1$ and Y are each as defined in claim 1), which comprises reacting a carboxylic acid of the general formula (V) or a reactive derivative thereof

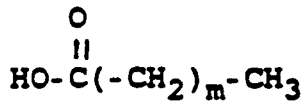

(V)

with an alcohol or amine of the general formula (VI),

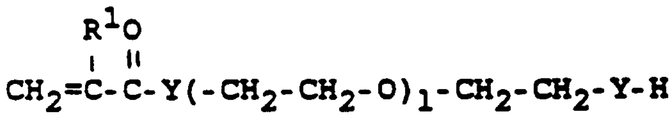

(VI)

preferably in the presence of a tertiary amine.

5. A homopolymer obtained by polymerization of a monomer as claimed in claim 1.

6. A copolymer obtained by polymerization of one or more monomers of the formulae (I) and/or (II) as claimed in claim 1 with or without further hydrophilic comonomers.

7. A process for preparing a polymer as claimed in claims 5 or 6 by polymerizing monomers in the presence of a free radical former.

8. A layer element comprising a film of at least one unimolecular layer of an amphiphilic compound on a solid base material, wherein the layer contains a polymer as claimed in claim 5 and/or 6.

9. A process for preparing a layer element as claimed in claim 8, which comprises dissolving at least one polymer as claimed in claims 5 and/or 6 in a volatile organic water-immiscible solvent, spreading the solution on the water/air interface, evaporating the solvent, compressing the resulting layer, and transferring the layer to a solid base material by the Langmuir-Blodgett technique.

10. The use of a film from a polymer as claimed in either of claims 5 and/or 6 for sealing surfaces and for reducing the friction between surfaces.

11. The use of the layer element as claimed in claim 8 for optical purposes.

12. The use of a film of a polymer as claimed in either of claims 5 and 6 for electrical insulation purposes.

## Revendications

1. Monomère amphiphile de formule générale (I) ou (II)

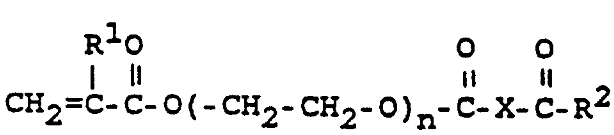

(I)

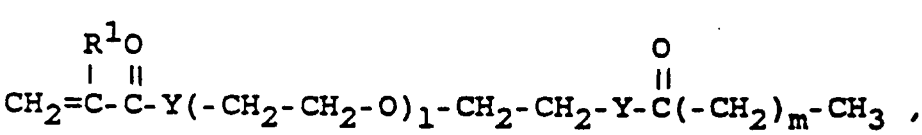

(II)

où
X = $(CH_2)_n$ ou $(CH_2\text{-}O\text{-}CH_2)_n$,
Y = O ou NH,

l est un nombre entier compris entre 0 et 10, m est un nombre entier compris entre 13 et 26, de préférence entre 13 et 21, n est un nombre entier compris entre 1 et 10,
$R^1$ = H, $CH_3$, Cl, CN, F ou Br, et
$R^2$ est un radical O-alkyle ou un radical NH-alkyle avec une chaîne alkyle d'au moins 8 atomes de carbone, de préférence n-alkyle avec 12 à 22 atomes de C.

**2.** Procédé pour la préparation d'un monomère de formule générale (I)

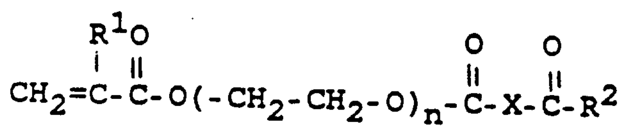
(I)

(X, n, $R^1$ et $R^2$ ayant la signification donnée dans la revendication 1),
caractérisé en ce qu'on fait réagir un acide dicarboxylique de formule générale (III)

HOOC-X-COOH (III)

ou un dérivé activé de cet acide dicarboxylique avec un alcool insaturé de formule générale (IV)

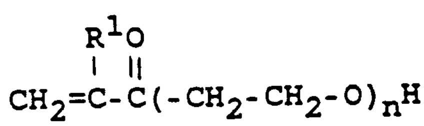
(IV)

et on fait réagir ultérieurement le monoester alors formé avec un alcool à longue chaîne $R^2H$, respectivement une amine primaire $R^2H$.

**3.** Procédé pour la préparation d'un monomère de formule générale (I), caractérisé en ce qu'on fait réagir d'abord un acide dicarboxylique de formule générale (III)

HOOC-X-COOH (III)

ou un dérivé activé de cet acide dicarboxylique avec un alcool à longue chaîne $R^2H$, respectivement une amine primaire $R^2H$, et on fait réagir ultérieurement le produit alors formé avec un alcool insaturé de formule générale (IV)

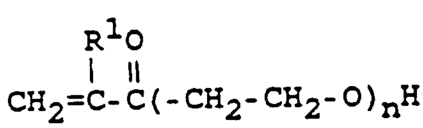
(IV)

X, $R^1$ et n ayant la signification donnée dans la revendication 1.

**4.** Procédé pour la préparation d'un monomère de formule générale (II)

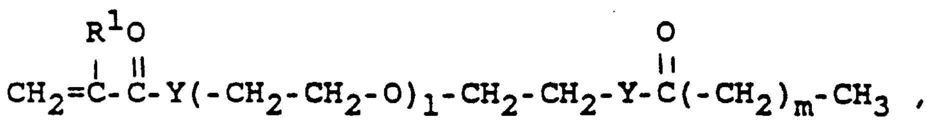
(II)

(l, m, $R^1$ et Y ayant la signification donnée dans la revendication 1)
caractérisé en ce qu'on fait réagir un acide carboxylique de formule générale (V) ou un dérivé réactif

d'acide carboxylique de cet acide carboxylique

$$HO-\overset{O}{\overset{\|}{C}}(-CH_2)_m-CH_3 \qquad (V)$$

avec un alcool, respectivement une amine, de formule générale (VI)

$$CH_2=\overset{R^1}{\overset{|}{C}}-\overset{O}{\overset{\|}{C}}-Y(-CH_2-CH_2-O)_l-CH_2-CH_2-Y-H \qquad (VI)$$

de préférence en présence d'une amine tertiaire.

5. Homopolymère obtenu par polymérisation d'un monomère selon la revendication 1.

6. Copolymère obtenu par polymérisation d'un ou plusieurs monomères de formules (I) et/ou (II) selon la revendication 1 ainsi qu'éventuellement d'un autre comonomère hydrophile.

7. Procédé pour la préparation de polymères selon les revendications 5 ou 6 par polymérisation de monomères, caractérisé en ce qu'on met en oeuvre la polymérisation en utilisant des formateurs de radicaux.

8. Elément stratifié d'un film déposé sur un support solide, composé d'au moins une couche monomoléculaire d'un composé amphiphile, caractérisé en ce que la couche contient un polymère selon la revendication 5 et/ou 6.

9. Procédé pour la préparation d'un élément stratifié selon la revendication 8, caractérisé en ce qu'on dissout au moins un polymère selon les revendications 5 et/ou 6 dans solvant organique volatil, non miscible à l'eau, on étale la solution sur l'interface eau/air, on comprime la couche obtenue après évaporation du solvant et on transfère sur un support solide à l'aide de la technique de Langmuir-Blodgett.

10. Utilisation d'un film d'un polymère selon l'une des revendications 5 et/ou 6 pour le traitement antireflet des surfaces et pour la réduction du frottement entre les surfaces.

11. Utilisation d'un élément stratifié selon la revendication 8 à des fins optiques.

12. Utilisation d'un film d'un polymère selon l'une des revendications 5 ou 6 pour isolation électrique.